# EUROPEAN PATENT SPECIFICATION

(11) **EP 2 817 410 B1**
(45) Date of publication and mention of the grant of the patent: **06.04.2016**
(21) Application number: 13704981.3
(22) Date of filing: 21.02.2013
(51) Int. Cl.: C12P 1/00

(54) **ADVANCED FERMENTATION CONTROL**
VERBESSERTE FERMENTATIONSSTEUERUNG
COMMANDE PERFECTIONÉE DE LA FERMENTATION

(30) Priority: 22.02.2012 EP 12156569
(43) Date of publication of application: 31.12.2014
(73) Proprietor: Novozymes A/S, 2880 Bagsvaerd (DK)
(72) Inventor: RIISGAARD, Frederik Kier, DK-4400 Kalundborg (DK); ANDERSSON, Jonas, S-212 32 Malmö (SE)
(86) International application number: PCT/EP2013/053444
(87) International publication number: WO 2013/124351

(56) References cited:
- SHANG ET AL: "High-cell-density fermentation for ergosterol production by Saccharomyces cerevisiae", JOURNAL OF BIOSCIENCE AND BIOENGINEERING, vol. 101, 2006, pages 38-41, XP028042338,
- ASAFF ET AL: "Carbon distribution and redirection of metabolism in Paecilomyces fumosoroseus during solid-state and liquid fermentation", PROCESS BIOCHEMISTRY, vol. 41, 2006, pages 1303-1310, XP027984155,
- HUANG ET AL: "The role of dissolved oxygen and function of agitation in hyaluronic acid fermentation", BIOCHEMICAL ENGINEERING JOURNAL, vol. 32, 2006, pages 239-243, XP028034550,
- BRUHEIM ET AL: "High-yield actinorhodin production in fed-batch culture by a Streptomyces lividans strain overexpressing the pathway-specific activator gene actII-ORF4", JOURNAL OF INDUSTRIAL MICROBIOLOGY & BIOTECHNOLOGY, vol. 28, 2002, pages 103-111, XP002676382,
- JOHANSEN ET AL: "Monitoring and control of fed-batch penicillin fermentation", COMPUTERS & CHEMICAL ENGINEERING, vol. 16 (Supplement 1), 1992, pages S297-S304, XP002676381,
- ERIKSEN ET AL: "On-line estimation of O2 production, CO2 uptake, and growth kinetics of microalgal cultures in a gas-tight photobioreactor", JOURNAL OF APPLIED PHYCOLOGY, vol. 19, 2007, pages 161-174, XP019502314,

## Description

### TECHNICAL FIELD

The present invention relates to a method of controlling the rate of carbon addition to a fermentation medium wherein the fermentation medium comprises a microorganism producing a compound of interest or the fermentation medium comprises a microorganism producing a microorganism of interest.

### BACKGROUND ART

Bacterial and fungal microorganisms are workhorses for industrial microbiology as they are used for the commercial production of many different therapeutics (e.g. penicillin and cephalosporin), pharmaceutical proteins (e.g. insulin), polysaccharides (e.g. hyaluronic acid), enzymes (e.g. proteases), and commodity chemicals (e.g. citric acid).

In industry, it is very common to use a fed batch fermentation process. A fed batch fermentation process is a process which is based on feeding of a growth limiting nutrient substrate to a culture. The growth limiting nutrient substrate is typically a carbohydrate.

Different strategies have been used in order to control the growth in a fed batch process. Control parameters such as DOT (dissolved oxygen tension), and pH (pHstat) are normally followed closely.

Shang et al. (2006) The society for Biotechnology Japan, discloses a study of fermentation for ergosterol production by *Saccharomyces cerevisiae.* Different parameters for controlling the fermentation were investigated and it was concluded that controlling glucose feeding rate in accordance with ethanol concentration were effective fed- batch methods for ergosterol production.

Bruheim et al. (2002) J Indus Microbiol. Biotechnol 28, 201-111 discloses fed-batch fermentation of Streptomyces lividans for production of actinorhodin. Glucose was fed to the fermenters in a rate to keep glucose concentration above 10g/l.

Johansen et al (1992), Comp. Chem Eng 16, 297-304, discloses an automized system for monitoring and control of fed-batch penicillin fermentations. The fermentations are carried out in a fed batch mode where glucose, ammonia and precursore is added either together or in separate feed streams.

The rate of carbohydrate addition in a fed batch fermentation, wherein a microorganism produces a compound of interest or the microorganism produces a microorganism of interest, is a highly critical process parameter. Overfeeding of the carbohydrate has been shown to lead to loss of batches or severely lowered productivity due to, e.g., overflow metabolism and production of side products.

The present invention presents a method for monitoring and controlling the microbial carbon utilization efficiency in a fermentation medium and thereby avoiding overfeeding.

### SUMMARY OF THE INVENTION

The present invention discloses a method of controlling the rate of carbon addition to a fermentation medium in a fed batch or a continuous fermentation medium comprising
measuring the C-moles lost to carbon dioxide;
measuring the C-moles added via the carbon addition;
calculating the carbon dioxide feed quotient, FQ, as C-moles carbon dioxide produced divided by C-moles carbon added over a time period; and
adjusting the rate of carbon addition depending on the FQ of interest, or
measuring the moles of consumed oxygen;
measuring the C-moles added via the carbon addition;
calculating the oxygen feed quotient, OQ, as moles oxygen consumed divided by C-moles carbon added over a time period; and
adjusting the rate of carbon addition depending on the OQ of interest,
wherein said fermentation medium comprises a microorganism producing a compound of interest or wherein said fermentation medium comprises a microorganism producing a microorganism of interest.

### BRIEF DECRIPTION OF DRAWINGS

The present invention is further illustrated by reference to the accompanying drawings, in which
Fig. 1 shows the normalized enzyme concentration in Batch X, Batch Y and Batch Z, wherein Batch X is a standard fermentation (DOT control); batch Y is a fermentation with FQ control;
and batch Z is a fermentation with OQ control.
Fig. 2 shows the normalized biomass concentration in Batch X, Batch Y and Batch Z.
Fig. 3 shows the FQ process values in Batch X (DOT control) and Batch Y (FQ control).
Fig. 4 shows the OQ process values in Batch X (DOT control) and Batch Z (OQ control).
Fig. 5 shows the nutrient feed rate in Batch X, Batch Y and Batch Z.
Fig. 6 shows the dissolved oxygen for Batch X, and the DOT set-point for Batch X (=20%).
Fig. 7 shows measured OQ according to Example 2. The OQ set-point was 0.3.
Fig. 8 shows feed rate of glucose regulated by the OQ controller according to Example 2.
Fig. 9 shows measured OQ according to Example 3. The OQ set-point was ramped from 0.45 at 0h to 0.6 at 70h.
Fig. 10 shows OQ controlled lactose feed rate according to Example 3.
Fig. 11 shows measured enzyme concentration with index 100 at 71 h according to Example 3.

### DETAILED DISCLOSURE OF THE INVENTION

It is known in the art that the rate of carbohydrate addition in, e.g., a *Bacillus* fed batch fermentation is a highly critical process parameter. Overfeeding of a substrate has been shown to lead to loss of batches or lowered carbon utilization efficiency.

This invention presents a method for monitoring and controlling the microbial carbon utilization efficiency in fermentation processes whereby it is possible to optimize the process without risking overfeeding. In particular, the present invention presents a method for monitoring and controlling the microbial carbon utilization efficiency in an aerobic and carbon limited fermentation whereby it is possible to optimize the process without risking overfeeding.

### Microorganisms

The microorganism producing or expressing the compound of interest according to the invention may be any microorganism known in the art that can be cultivated in a fermentor.

The microorganism according to the invention may be a bacterial strain, e.g., a Gram-positive strain such as a *Bacillus, Clostridium, Enterococcus, Geobacillus, Lactobacillus, Lactococcus, Oceanobacillus, Staphylococcus, Streptococcus,* or *Streptomyces* strain, or a Gram-negative strain such as a *Campylobacter, Escherichia, Flavobacterium, Fusobacferium, Helicobacter, Ilyobacter, Neisseria, Pseudomonas, Salmonella,* or *Ureaplasma strain.*

In one aspect, the strain is a *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* or *Bacillus thuringiensis* strain; in particular the strain is a *Bacillus licheniformis* strain.

In another aspect, the strain is a *Streptococcus equisimilis, Streptococcus pyogenes, Streptococcus uberis,* or *Streptococcus equi* strain.

In another aspect, the strain is a *Streptomyces achromogenes, Streptomyces avermitilis, Streptomyces coelicolor, Streptomyces griseus,* or *Streptomyces lividans* strain.

The microorganism may be a fungal strain. For example, the strain may be a yeast strain such as a *Candida, Kluyveromyces, Pichia, Saccharomyces, Schizosaccharomyces,* or *Yarrowia* strain; or a filamentous fungal strain such as an *Acremonium, Agaricus, Alternaria, Aspergillus, Aureobasidium, Botryospaeria, Ceriporiopsis, Chaetomidium, Chrysosporium, Claviceps, Cochliobolus, Coprinopsis, Coptotermes*, *Corynascus, Cryphonectria, Cryptococcus, Diplodia, Exidia, Filibasidium, Fusarium, Gibberella, Holomastigotoides, Humicola, Irpex, Lentinula, Leptospaeria, Magnaporthe, Melanocarpus, Meripilus, Mucor, Myceliophthora, Neocallimastix, Neurospora, Paecilomyces, Penicillium, Phanerochaete, Piromyces, Poitrasia, Pseudoplectania, Pseudotrichonympha, Rhizomucor, Schizophyllum, Scytalidium, Talaromyces, Thermoascus, Thielavia, Tolypocladium, Trichoderma, Trichophaea, Verticillium, Volvariella,* or *Xylaria* strain.

In another aspect, the strain is an *Acremonium cellulolyticus, Aspergillus aculeatus, Aspergillus awamori, Aspergillus foetidus, Aspergillus fumigatus, Aspergillus japonicus, Aspergillus nidulans, Aspergillus niger, Aspergillus oryzae, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola grisea, Humicola insolens, Humicola lanuginosa, Irpex lacteus, Mucor miehei, Myceliophfhora thermophila, Neurospora crassa, Penicillium funiculosum, Penicillium purpurogenum, Phanerochaete chrysosporium, Thielavia achromatica, Thielavia albomyces, Thielavia albopilosa, Thielavia australeinsis, Thielavia fimeti, Thielavia microspora, Thielavia ovispora, Thielavia peruviana, Thielavia setosa, Thielavia spededonium, Thielavia subthermophila, Thielavia terrestris, Trichoderma harzianum, Trichoderma koningii, Trichoalerma longibrachiatum, Trichoderma reesei,* or *Trichoderma viride* strain.

In another aspect, the strain is a yeast strain, e.g.,a *Saccharomyces carlsbergensis, Saccharomyces cerevisiae, Saccharomyces diastaticus, Saccharomyces douglasii, Saccharomyces kluyveri, Saccharomyces norbensis,* or *Saccharomyces oviformis* strain.

Strains of these species are readily accessible to the public in a number of culture collections, such as the American Type Culture Collection (ATCC), Deutsche Sammlung von Mikroorganismen und Zellkulturen GmbH (DSMZ), Centraalbureau Voor Schimmelcultures (CBS), and Agricultural Research Service Patent Culture Collection, Northern Regional Research Center (NRRL).

### Microorganism producing a microorganism of interest

The product of the fermentation may be a microorganism, e.g., the microorganism of interest may be a yeast or a bacterial strain, in particular a *Saccharomyces* strain or a *Bacillus* strain or a *Bacillus* spore.

### Compound of interest

The compound of interest according to the invention may be an antibiotic such as penicillin or cephalosporin or erythromycin, or a commodity chemical such as citric acid.

The compound of interest may also be a polysaccharide such as hyaluronic acid, or a therapeutic polypeptide such as insulin, or a peptide, or a protein such as an enzyme.

A preferred peptide according to this invention contains from 2 to 100 amino acids; preferably from 10 to 80 amino acids; more preferably from 15 to 60 amino acids; even more preferably from 15 to 40 amino acids.

In a preferred embodiment, the compound of interest is an enzyme, in particular a hydrolase (class EC 3 according to Enzyme Nomenclature; Recommendations of the Nomenclature Committee of the International Union of Biochemistry).

In a particular preferred embodiment the following hydrolases are preferred: Proteases: Suitable proteases include those of animal, vegetable or microbial origin. Microbial origin is preferred. Chemically modified or protein engineered mutants are included. The protease may be an acid protease, a serine protease or a metallo protease, preferably an alkaline microbial protease or a trypsin-like protease. Examples of alkaline proteases are subtilisins, especially those derived from *Bacillus,* e.g., subtilisin Novo, subtilisin Carlsberg, subtilisin 309, subtilisin 147 and subtilisin 168 (described in WO 89/06279). Examples of trypsin-like proteases are trypsin (e.g. of porcine or bovine origin) and the *Fusarium* protease described in WO 89/06270 and WO 94/25583.

Examples of useful proteases are the variants described in WO 92/19729, WO 98/20115, WO 98/20116, and WO 98/34946, especially the variants with substitutions in one or more of the following positions: 27, 36, 57, 76, 87, 97, 101, 104, 120, 123, 167, 170, 194, 206, 218, 222, 224, 235 and 274.

Preferred commercially available protease enzymes include ALCALASE™, SAVINASE™, PRIMASE™, DURALASE™, ESPERASE™, RELASE™ and KANNASE™ (Novozymes A/S), MAXATASE™, MAXACAL™, MAXAPEM™, PROPERASE™, PURAFECT™, PURAFECT OXP™, FN2™, and FN3™ (Genencor International Inc.). Lipases: Suitable lipases include those of bacterial or fungal origin. Chemically modified or protein engineered mutants are included. Examples of useful lipases include lipases from *Humicola* (synonym *Thermomyces*), e.g. from *H*. *lanuginosa* (*T. lanuginosus*) as described in EP 258 068 and EP 305 216 or from *H*. *insolens* as described in WO 96/13580, a *Pseudomonas* lipase, e.g. from *P. alcaligenes* or *P. pseudoalcaligenes* (EP 218 272), *P. cepacia* (EP 331 376), *P. stutzeri* (GB 1,372,034), *P. fluorescens, Pseudomonas sp.* strain SD 705 (WO 95/06720 and WO 96/27002), *P*. *wisconsinensis* (WO 96/12012), a *Bacillus* lipase, e.g. from *B. subtilis* (Dartois et al. (1993), Biochemica et Biophysica Acta, 1131, 253-360), *B. stearothermophilus* (JP 64/744992) or *B. pumilus* (WO 91/16422).

Other examples are lipase variants such as those described in WO 92/05249, WO 94/01541, EP 407 225, EP 260 105, WO 95/35381, WO 96/00292, WO 95/30744, WO 94/25578, WO 95/14783, WO 95/22615, WO 97/04079 and WO 97/07202.

Preferred commercially available lipase enzymes include LIPOLASE™, LIPOLASE ULTRA™ and LIPEX™ (Novozymes A/S).
Amylases: Suitable amylases (alpha and/or beta) include those of bacterial or fungal origin. Chemically modified or protein engineered mutants are included. Amylases include, for example, alpha-amylases obtained from *Bacillus,* e.g. a special strain of *B*. *licheniformis,* described in more detail in GB 1,296,839.

Examples of useful amylases are the variants described in WO 94/02597, WO 94/18314, WO 96/23873, WO 97/43424, and WO 01/66712, especially the variants with substitutions in one or more of the following positions: 15, 23, 105, 106, 124, 128, 133, 154, 156, 181, 188, 190, 197, 202, 208, 209, 243, 264, 304, 305, 391, 408, and 444.

Commercially available amylases are DURAMYL™, TERMAMYL™, FUNGAMYL™, NATALASE™, TERMAMYL LC™, TERMAMYL SC™, LIQUIZYME-X™ and BAN™ (Novozymes A/S), RAPIDASE™ and PURASTAR™ (from Genencor International Inc.). Cellulases: Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium,* e.g. the fungal cellulases produced from *Humicola insolens, Myceliophthora thermophila* and *Fusarium oxysporum* disclosed in US 4,435,307, US 5,648,263, US 5,691,178, US 5,776,757 and WO 89/09259.

Especially suitable cellulases are the alkaline or neutral cellulases having colour care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and PCT/DK98/00299.

Commercially available cellulases include CELLUZYME™, CAREZYME™ , and CAREZYME CORE™ (Novozymes A/S), CLAZINASE™, and PURADAX HA™ (Genencor International Inc.), and KAC-500(B)™ (Kao Corporation).
Pullulanases: Pullulanases include those of bacterial or fungal origin. Chemically modified or protein engineered mutants are included.

The pullulanase according to the present invention is preferably a pullulanase from e.g. *Pyrococcus* or *Bacillus,* such as *Bacillus acidopullulyticus,* e.g., the one described in Kelly et al., 1994, FEMS Microbiol. Letters 115: 97-106; or a pullulanase available from Novozymes A/S such as Promozyme™.

The pullulanase may also be from *Bacillus naganoencis,* or *Bacillus deramificans,* e.g., such as derived from *Bacillus deramificans* (US Patent 5,736,375).
Oxidoreductases: Oxidoreductases include those of bacterial or fungal origin. Chemically modified or protein engineered mutants are included. Oxidoreductases include include peroxidases, and oxidases such as laccases, and catalases.

Other preferred hydrolases are carbohydrolases including MANNAWAY™. Other preferred enzymes are transferases, lyases, isomerases, and ligases.

### Fermentations

The present invention may be useful for any fed batch or continuous fermentation in any scale wherein it is possible to measure the gas composition, the gas flow, and the carbohydrate flow.

Accordingly, the present invention may be useful for any fermentation having a culture medium in micro scale and up to any fermentation having a culture medium in industrial scale, e.g., typically of from 1000 liters to 500.000 liters.

The microorganism may be fermented by any method known in the art. The fermentation medium may be a complex medium comprising complex nitrogen and/or carbon sources, such as soybean meal, soy protein, soy protein hydrolysate, cotton seed meal, corn steep liquor, yeast extract, casein, casein hydrolysate, potato protein, potato protein hydrolysate, molasses, and the like. The fermentation medium may be a chemically defined media, e.g. as defined in WO 98/37179.

The fermentation may be performed using carbon limited conditions. Carbon limited conditions mean that the growth of microorganism is controlled by the addition of the carbon source.

### Carbon sources

The present invention may be useful for any carbon source wherein it is possible to measure the metabolizable carbon.

A carbohydrate or carbon source selected from the group consisting of glucose, sucrose, fructose, maltose, lactose, maltulose, mannose, glycerol, and galactose is preferred; in particular a carbon source selected from the group consisting of glucose, sucrose, lactose, glycerol and maltose is preferred.

### Carbon Dioxide Feed quotient (FQ)

The utilization efficiency is measured as the quotient between C-moles lost to carbon dioxide (CO2) and C-moles added via the carbohydrate feeding (named Carbon Dioxide Feed Quotient or FQ), wherein
1 C-mole = mole x Nc,
wherein Nc is the number of carbon atoms in the molecule and "mole" is the amount of substance in moles.

This means that 1 mole of glucose equals 6 C-moles of glucose.

The carbon dioxide feed quotient (FQ) is monitored on-line by the use of a carbon balance on the fermentation tank. The lost CO2 is measured over a defined time interval by a gas analyzer on the incoming gas and the off-gas combined with measurement of the air flow rate.

The C-moles carbohydrate dosed into the tank over a defined time interval in the form of a carbohydrate solution is measured by the volumetric feeding rate combined with data on carbohydrate type and concentration measured by, e.g., refractive index (RI).

A suitable time period will typically be in the range of from 1 sec. to 20 minutes; in particular of from 1 minute to 15 minutes.

Over this time period the FQ is calculated by dividing the C-moles lost to CO2 with the added C-moles carbohydrate.

FQ is logged by a process computer and compared to a user defined FQ set-point from which a new set-point for the carbohydrate feeding rate is calculated and automatically adjusted by use of, e.g., a PI controller.

The user defined set-point may be defined by running a series of fermentations and thereby finding the optimal FQ. The optimal FQ is typically when the FQ is ≥ 0.2; 0.2; in particular when the FQ is in the range of from 0.2 to 0.75; e.g., in the range of from 0.2 to 0.7; e.g., in the range of from 0.25 to 0.75.

The fermentation may be performed by using a constant FQ during the whole fermentation; or it may be ramped. If it is ramped it may typically start with an FQ of around 0.5 and end with an FQ of around 0.7. The FQ may typically start with a low value in order to promote enough growth.

The implementation of an FQ controller is fairly straightforward and can easily be done with the standard instrumentation currently available in the art.

A basic assumption for this invention is that a critical FQ value exists for each type of fermentation. Below this critical FQ value overfeeding occurs, and the associated overflow metabolism starts.

The idea of the invention is therefore to control the carbohydrate feeding rate on-line in such a way that FQ is maintained at the set-point and above the critical FQ value (e.g. 0.5) and thereby automatically avoid overfeeding but at the same time promote the necessary growth and product formation.

It is possible to avoid overfeeding during the fermentation: If FQ is lower than the pre-defined set-point then the fermentation is overdosed (not enough carbon is transformed into CO2 - some is left not metabolized or is converted into overflow metabolites). With the FQ controller implemented, an FQ that is below the current set-point will automatically lead to a decrease of the carbohydrate feed rate and overfeeding will therefore be avoided.

Maximize feeding: If FQ is above the pre-defined set-point then the fermentation can handle more feed and the feed rate is increased.

Maximize yield of compound of interest per fermentation tank: An optimal FQ exists and the present invention allows for optimization on this parameter.

Experiments run with the FQ controller have shown that it is possible to use the invention in various scales.

The FQ control may be combined with other standard control regimes like DOT control to ensure aerobic conditions: The feeding rate will in that case only increase if DOT and FQ are high whereas the feeding rate will decrease if just DOT or FQ is low.

### Oxygen Feed quotient (OQ)

The utilization efficiency is measured as the quotient between moles consumed 02 and C-moles carbon added via the sugar feeding (named Oxygen Feed Quotient or OQ).

The oxygen feed quotient (OQ) is monitored on-line by the use of an elemental balance on the fermentation tank. The consumed O is measured over a defined time interval by a gas analyzer as the difference in oxygen content in the incoming gas and the off-gas combined with measurement of the air flow rate.

The C-moles carbohydrate dosed into the tank over a defined time interval in the form of a carbohydrate solution is measured by the volumetric feeding rate combined with data on carbohydrate type and concentration measured by, e.g., refractive index (RI).

A suitable time period will typically be in the range of from 1 sec. to 20 minutes; in particular of from 1 minute to 15 minutes.

Over this time period the OQ is calculated by dividing the consumed mol 02 with the added C-mole carbohydrate.

OQ is logged by a process computer and compared to a user defined OQ set-point from which a new set-point for the carbohydrate feeding rate is calculated and automatically adjusted.

The user defined set-point may be defined by running a series of fermentations and thereby finding the optimal OQ. The optimal OQ is typically when the OQ is ≥ 0.2; in particular the OQ will be in the range of from 0.2 to 0.75; e.g., in the range of from 0.2 to 0.7; e.g., in the range of from 0.25 to 0.75.

The fermentation may be performed by using a constant OQ during the whole fermentation; or it may be ramped. If it is ramped it may typically start with an OQ of 0.45 and end with an OQ of 0.65.

The implementation of an OQ controller is fairly straightforward and can easily be done with the standard instrumentation currently available in the art.

A basic assumption for this invention is that a critical OQ value exists for each fermentation. Below this critical OQ value the microorganism starts to become overdosed.

Data indicate that batches with OQ values below 0.45 suffer from being overdosed. The idea is therefore to control the carbohydrate feeding rate on-line in such a way that OQ is maintained at the set-point and above the critical OQ value (e.g. 0.45) and thereby automatically avoid overfeeding.

Automatic avoidance of overfeeding during the fermentation: if OQ is lower than the pre-defined set-point then the fermentation is overdosed (there is no balance between metabolized carbon and oxygen consumed). With the OQ controller implemented an OQ that is below the current set-point will automatically lead to a decrease of the carbohydrate feed rate and overfeeding will therefore be avoided.

Maximize feeding: if OQ is above the pre-defined set-point then the fermentation can handle more feed and the feed rate is increased.

Maximize yield of compound of interest per fermentation tank: An optimal OQ exists and the present invention allows for optimization on this parameter.

Experiments run with the OQ controller have already shown that it is possible to reach the same activity levels in pilot and in production.

The OQ control may be combined with other standard control regimes like DOT control: The feeding rate will in that case only increase if DOT and OQ are high whereas the feeding rate will decrease if just DOT or OQ is low.

The FQ control and the OQ control are not normally combined. Depending of the fermentation of interest - trials will be performed - and the control that gives the best results will be chosen.

### Recovery of the compound of interest

A further aspect concerns the downstream processing of the fermentation broth. After the fermentation process is ended, the compound of interest may be recovered from the fermentation broth, using standard technology developed for the compound of interest.

The relevant downstream processing technology to be applied depends on the nature of the compound of interest.

A process for the recovery of a compound of interest from a fermentation broth will typically (but is not limited to) involve some or all of the following steps:
1) pre-treatment of broth (e.g. flocculation)
2) removal of cells and other solid material from broth (primary separation)
3) filtration
4) concentration
5) stabilization and standardization.

Apart from the unit operations listed above, a number of other recovery procedures and steps may be applied, e.g., pH-adjustments, variation in temperature, crystallization, treatment of the solution comprising the compound of interest with active carbon, use of chromatography, and use of various adsorbents.

The invention is further illustrated in the following example which is not intended to be in any way limiting to the scope of the invention as claimed.

### Example 1

Fed-batch fermentation of *Bacillus licheniformis* expressing a pullulanase with FQ and OQ control implemented to increase process robustness and enzyme production

### Materials and Methods

### Strain and Growth Conditions

In all experiments a *Bacillus licheniformis* strain expressing a pullulanase (*Bacillus acidopullulyticus* pullulanase - pullulanase 3 described in WO 09/075682) was used.

For storage, the culture was propagated in LB broth at 37°C and glycerol was added to a final concentration of 15% (w/v).
The suspension was divided into 1 ml cryotubes and maintained at below -70°C.
For inoculation, 0.1 ml of the thawed cryotube suspension was spread on SSB4 medium slants with the following composition: Soy peptone SE50MK 10 g/l, Sucrose 10 g/l, K2HPO4 2 g/l, Na2HPO4•2H2O 5 g/l, Vitamins (Thiamin-hydrochlorid 11,4 mg/l, Riboflavin 0,95 mg/l, Nicotinic amide 7,8 mg/l, Calcium D-pantothenate 9,5 mg/l, Pyridoxal-HCl 1,9 mg/l, D-biotin 0,38 mg/l, Folic acid 2.9 mg/l), Trace metals (MnSO4, H2O 9.8 mg/l, FeSO4•7H2O 39,3 mg/l, CuSO4•5H2O 3,9 mg/l, ZnSO4•7H2O 8,2 mg/l), Agar 25 g/l. Deionized water was used, and pH adjusted to pH 7.3 to 7.4 with NaOH, after which the medium was sterilized.
The cells were grown on the agar for 24 h at 37°C and then re-suspended in sterilized deionized water and used to inoculate the seed fermentor with a nominal volume of 20 I.

In the seed fermentor, 15 l of complex growth medium was used with the following composition (after sterilization): Soy grits 110 g/l, Na2HPO4•2H2O 5 g/l. Potable water was used to prepare the medium. Prior to inoculation the pH was adjusted to 7.0 with ammonia water or H3PO4. During fermentation aeration was controlled at 1.5 vvm, temperature controlled at 37°C, back pressure controlled at 1 atm. and agitation controlled at 1000 rpm. The seed culture was stopped when the total oxygen uptake reached the arbitrary value of 2.6 mol. The main fermentors were inoculated with 10 % (w/v) each of the seed culture.

### Fermentation conditions

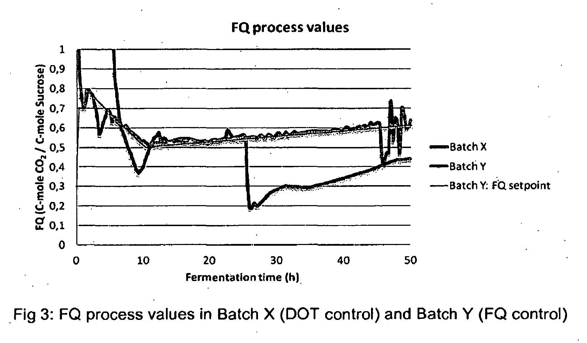

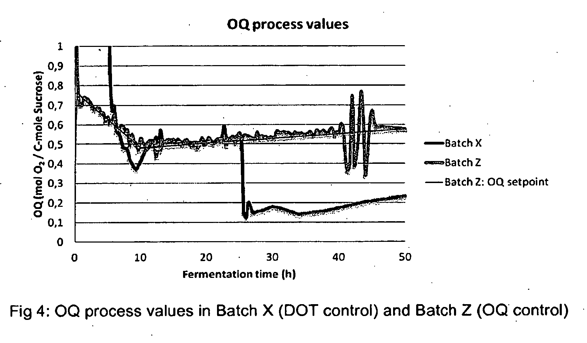

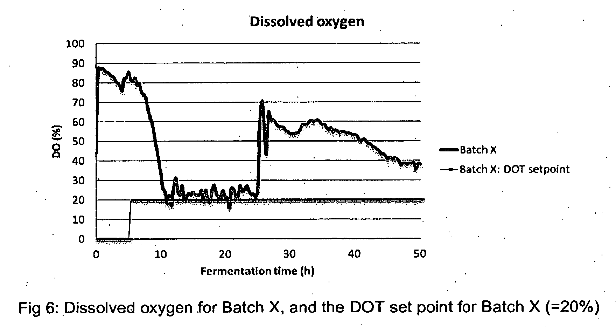

Three fermentation batches (Batch X, Batch Y and Batch Z) with a starting volume of 8 I were inoculated from the seed fermentor.
The fermentation medium contained (before inoculation and after sterilization): MgSO4•7H2O 4 g/l, K2HPO4 7 g/l, Na2HPO4•H2O 7 g/l, (NH4)2SO4 4 g/l, K2SO4 5 g/l, Citric acid 0.78 g/l, Vitamin (D-biotin 1.25 mg/l), Trace metals (MnSO4, H2O 39.2 mg/l; FeSO4, 7H2O 157 mg/l; CuSO4, 5H2O 15.6 mg/l; ZnSO4, 7H2O 32,8 mg/l), Antifoam (SB2121) 1.25 ml/l, Sucrose 10 g/l (only in Batch X). Potable water was used to prepare the medium. Before inoculation the pH was adjusted to 6.0 with ammonia water or H3PO4. During cultivation the temperature was controlled at 37°C, the minimum pH maintained at 6.0 (ammonia water), the maximum pH controlled at 7.0 (H3PO4), the agitation set to 800 rpm (0 h) and ramped to 1000 rpm (1 h), the aeration controlled at 1.5 vvm and the back pressure controlled at 1 atm.
A 650 g/l sucrose solution was used as feed medium which was prepared with potable water and sterilized.
Three fed-batch fermentation processes were conducted as described below:
**Batch X:** Initial batch phase followed by fed batch with dissolved oxygen (DOT) controlled nutrient feed (PI control). The feed was started after a drop in carbon dioxide evolution rate which signalled depletion of initial sugar. The set-point for the DOT control was set to 20% (see Fig. 6).
**Batch Y:** Fed batch with FQ controlled (PI control) nutrient feed from start of fermentation. The set-points for the controller followed a predefined profile (see Fig 3).
**Batch Z:** Fed batch with OQ controlled (PI control) nutrient feed from start of fermentation. The set-points for the controller followed a predefined profile (see Fig. 4).

In all three batches, media components and media concentrations were identical except that sucrose was added as an initial carbon source to Batch X, as is generally accepted standard practice for this type of process. This is not required for the new process types, Y & Z, and consequently the feed was initiated directly after inoculation.

Throughout the fermentation processes triplicate samples for dry weight measurement and single samples for enzyme activity analysis and at-line pH were taken out daily. The CV of the activity analysis is less than 3.2%. All control loops were running in the distributed control system DeltaV (® Emerson Electric Company), and data acquisition was done on-line in the same system for all parameters. Similarly, the FQ, OQ and DOT nutrient feed controllers were implemented in DeltaV (® Emerson Electric Company). Off gas measurement for CO₂ and O₂ was done on-line by a mass spectrometer.

### Results

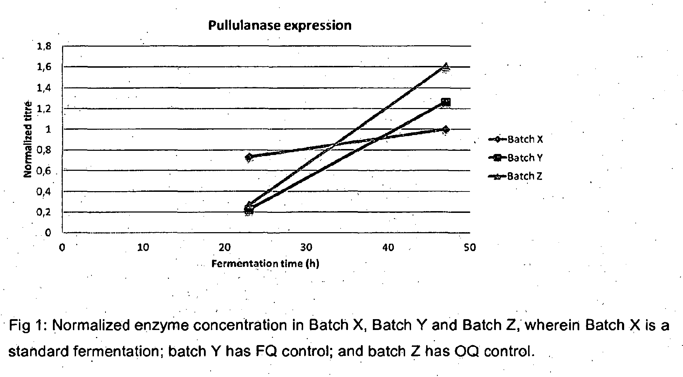

The batches using FQ control (Batch Y) and OQ control (batch Z) reached a significantly higher enzyme titre than the batch using a standard fermentation nutrient feed method known in the art (Batch X). At the end of the fermentation process Batch Y had an enzyme titre which was 20% above the highest level in Batch X. Similarly, Batch Z reached 60% above the highest level in Batch X (see FIG. 1).

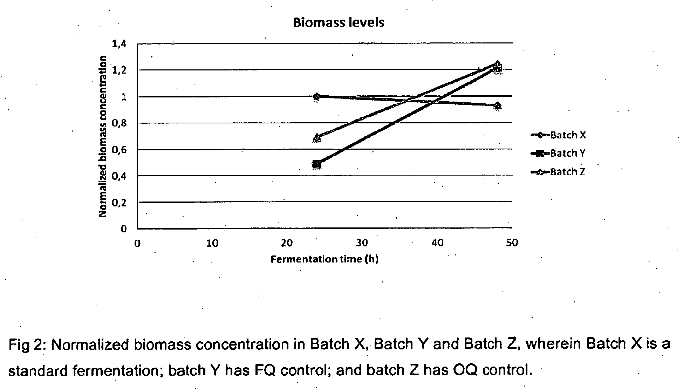

In addition the fermentation processes controlled by FQ (Batch Y) and OQ (Batch Z) facilitated a much higher biomass level. At the end of the fermentation process, both Batch Y and Batch Z had a 20% higher biomass level than the highest level obtained in Batch X (see FIG. 2).

The control of FQ and OQ by the automatic manipulation of the nutrient feed rate was very accurate throughout the fermentation processes and the process values were thereby closely aligned to the predefined set-point profile (see FIG. 3 and FIG. 4).

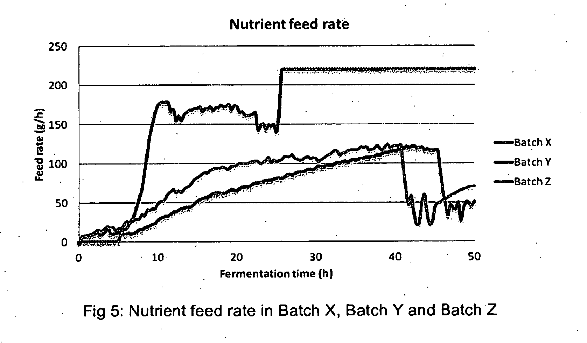

At the end of the process, after approximately 45 h in Batch Y and approximately 40 h in Batch Z, a sudden drop in FQ and OQ occurred. This drop signals that the fermentation batches suddenly reduce their rate of metabolism and therefore are in risk of being overfed. However, the FQ and OQ controllers adjust the feed rate and automatically reduce it to a lower level, appropriate for the new culture conditions (see FIG. 5).
Consequently, control of the fermentation process is maintained and the FQ and OQ parameters were, after some fluctuations, returned to the set-point profile (see FIG. 3 and FIG. 4, respectively).

In the fermentation batch running the standard practice method (Batch X) the DOT level is accurately controlled by the nutrient feed rate until 25 h into the fermentation.
At this time a metabolic problem associated with overfeeding occurs, and DOT suddenly rises. This is similar to what was seen in Batch Y and Batch Z. This problem occurred roughly 20 h earlier in Batch X, compared to Batch Y and Batch Z. Since the standard practice method used a DOT controlled feed rate the response of the DOT rise was for the controller to increase the feed rate (FIG. 5). This continued until the feed rate hit the maximum capacity of the feed pump and for the rest of the process the DOT was above the process set-point. Thus, the responses to the physiological condition for the different control strategies are fundamentally different: After overfeeding, FQ and OQ controllers reduce the feed rate automatically and maintain process control as FQ and OQ are returned to their respective set-points (FIG. 3 and FIG. 4, respectively), whereas DOT control increases the feed rate and reaches a state where the process can no longer be controlled (see Fig. 6).

### Conclusions

A standard fermentation method (Batch X) was used as comparison for evaluating the two control strategies; FQ and OQ in Batch Y and Z, respectively.
From the results it is clear that the use of these new control strategies facilitate a more robust fermentation process where process control can be maintained throughout the fermentation. Secondly, the physiological change is much delayed in the batches controlled by FQ and OQ control and occurs roughly 20 h later than in Batch X. Finally, the FQ and OQ controlled batches reach a significantly higher end titre and biomass level than the process running the standard method known in the art.

### Example 2

Fed-batch fermentation of *Saccharomyces cerevisiae* with OQ controlled glucose feed rate

### Materials and Methods

### Strain and Growth Conditions

In the experiment, a *Saccharomyces cerevisiae* yeast with the commercial name EthanolRed® was used.

Storage: the granulated dry yeast was maintained at 5°C in a plastic envelope.

Selection of a single colony: a granule was spread on a YPD agar and incubated for 3 days at 30°C. The YPD agar contained: Yeast extract 10 g/L, Bacto Peptone 20 g/L, Dextrose 30 g/L, Agar 20 g/L.

Propagation on YPD agar: a single colony was picked from the YPD agar and dissolved in M9 buffer. The cell containing buffer solution was then spread over a new YPD agar (ø=11 cm) and incubated for 3 days at 30°C. All cells from the ø=11 cm YPD agar were scraped off using 100 ml 0.1% Tween solution to obtain 100 ml inoculum which was then transferred to a seed fermentor.

### Seed fermentation conditions

A seed fermentor with a nominal volume of 20 liters and containing 10 liters of sterile growth medium was inoculated with 50 ml of the inoculum.

The seed growth medium contained before inoculation: Glucose 27.3 g/L, Bacto Peptone 20 g/L, Bacto Yeast extract 10 g/L, H2SO4 (96%) 2 ml/l, MgSO4•7H2O 9 g/L, K2HPO4 14.3 g/L, KH2PO4 8 g/L, (NH4)2SO4 1.3 g/L, trace metals (ZnSO4•7H2O 37 mg/L, CuSO4•5H2O 18 mg/L, FeSO4•7H2O 177 mg/L, MnSO4•1H2O 107 mg/L), H3PO4 0.6 g/L, Dowfax 63n10 1 ml/l, Vitamin solution (m-inositol 1.96 g/L, Cholinchlorid 0.98 g/L, Thiaminchlorid 0.196 g/L, Pyridoxin (B6 vitamin) 0.196 g/L, Niacinamid (Nicotinamid) 0.196 g/L, d-Ca-Panthotenat 0.147 g/L, D-Biotin 0.0098 g/L, Folic acid 0.0098 g/L.). pH was adjusted to 5.5 by H3PO4 before sterilisation. Vitamin solution and glucose solution (66 % w/w) were sterile filtered into the batch after sterilization of the seed mix. Potable water was used to prepare the medium. Before inoculation the pH was adjusted to 5.5 with ammonia gas (NH3).

During seed fermentation, aeration was maintained at 1.2 VVM, the temperature was controlled at 32°C, back pressure controlled at 1.3 bar, and agitation was ramped up from 600 RPM at 0 hours to 900 RPM at 6 hours and maintained at 900 RPM throughout. The seed was stopped after 24h and app. 100 ml transferred to each main fermentor.

### Main fermentation conditions

A main fermentor with a nominal volume of 20 liters and a starting volume of 5 liters was inoculated with 100 ml from the seed fermentor. The fermentation medium contained before inoculation: the same as for seed except a low glucose concentration of 5.3 g/L.

During cultivation the temperature was controlled at 32°C, the agitation was ramped up from 600 RPM at 0 hours to 900 RPM at 6 hours and maintained at 900 RPM throughout, the aeration controlled at 12 L/min and the back pressure controlled at 1.3 bar. A 660 g/l glucose solution was used as feed medium which was prepared with potable water and sterilized. The minimum pH was maintained at 5.5 with NH3, and DOT was never below 200 mmHg (app. 40% of saturation). Glucose feed was OQ controlled (PI control) from the start of fermentation with an OQ set-point of 0.3.

All control loops were running in the control system Procos II, and data acquisition was done on-line in the same system for all parameters. Similarly, the OQ nutrient feed controller was implemented in Procos II. Off gas measurement for CO₂ and O₂ was done on-line by a photo-acoustic gas analyzer.

### Results

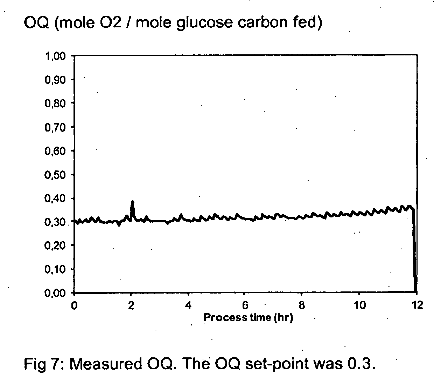

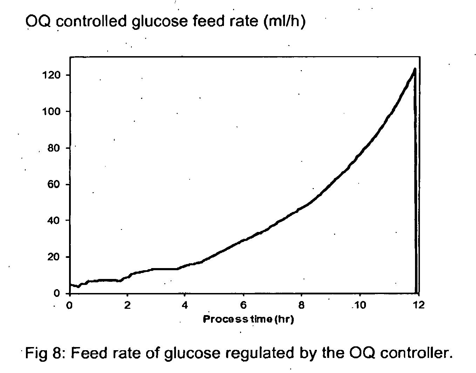

The fermentation had a batch phase from 0-4 hours where surplus glucose (5.3 g/L) from the main mix was being consumed. After this initial phase, from where the culture was glucose limited, the OQ controller controlled OQ at the set-point of 0.3. There was a minor increase (0.05) above the OQ set-point towards the end (see Fig. 7) which could be explained by a too conservative tuning of the PI parameters in the OQ controller. Nevertheless, the glucose feed rate increased according to the natural exponential growth of the yeast culture (see Fig. 8).

### Conclusions

It has been shown that an OQ controller can automatically adjust the glucose feed rate according to the needs and limitations during fermentation of *Saccharomyces cerevisiae* and thereby optimizing the growth rate without risking overflow metabolism.

### Example 3

### Fed-batch fermentation of Trichoderma reesei with OQ controlled lactose feed rate

### Materials and Methods

### Strain and Growth Conditions

In the experiment a non-GMO *Trichoderma reesei* producing cellulytic enzymes was used.

Storage: The vials were stored at - 80°C.

Propagation on PDA agar: Spores were produced during 10-16 days at 30°C in a 50 ml M-tube with a solidified agar slant. Spores were harvested by scraping off the spores with a syringe and 10 ml transfer buffer (M9-buffer). 3 ml of the obtained suspension was then aseptically transferred to a seed fermentor.

### Seed fermentation conditions

A seed fermentor with a nominal volume of 20L and containing 10 kg sterile growth medium was inoculated with 3 mL of the inoculum. The seed fermentation consisted of a batch phase of 70h.

The seed growth medium contained before inoculation:
Sucrose 50 g/L, corn steep powder 6.25 g/L, KH2PO4 4 g/L, (NH4)2SO4 6.35 g/L, MgSO4,7H2O 2 g/L, CaCl2 0.53 g/L, antifoam oil (Dowfax 63n10) 1.5 g/L, FeSO4,7H2O 0.0025 g/L, MnSO4,H2O 0.0016 g/L, ZnSO4,7H2O 0.0014 g/L. pH of the growth medium was 4.88 before sterilization. After sterilization pH was adjusted to 5.0 with NH3.

During the batch phase, pH decreased and at 70h pH had decreased to 4.2 which was the transfer signal. DOT was maintained by aeration of 8 L/min, back pressure of 0.5 bar and agitation of 600-750 rpm. Temperature was 25°C. 1.2 kg of the seed culture was transferred to a main fermentor.

### Main fermentation conditions

A main fermentor with a nominal volume of 20L and containing 10 kg of sterile growth medium was inoculated with 1.2 kg of seed culture.

The main fermentation medium contained before inoculation:
Corn steep powder 6.25 g/L, KH2PO4 4 g/L, (NH4)2SO4 6.35 g/L, MgSO4,7H2O 2 g/L, CaCl2 0.53 g/L, antifoam oil (Dowfax 63n10) 1.5 g/L, FeSO4,7H2O 0.0025 g/L, MnSO4,H2O 0.0016 g/L, ZnSO4,7H2O 0.0014 g/L. pH of the growth medium was 5.08 before sterilization. After sterilization pH was adjusted to 4.5 with H3PO4.

During fermentation the temperature was controlled at 25°C, the agitation was 950-1100 RPM, the aeration 0,8-1,2 vvm and the back pressure 1.3 bar. A 20 % w/w lactose solution was used as feed medium. pH was regulated at 4.5 with NH3 gas. DOT was never below the minimum set-point of 60 mmHg. Lactose feed was started right after inoculation, and it was OQ controlled with a ramped OQ set-point of 0.45 at 0h and 0.6 at 70h. Lactose feed was added by a peristaltic pump having a fixed rpm of 1.5 corresponding to a max feed rate of 152 ml/h. Average feed rate, as commanded by the OQ regulator, was achieved by automatic regulation of the pulse time within a given cycle time of 60 seconds. Antifoam oil (P2000) was added pre-emptively to the fermentor in pulses of 1.5 ml every 3 hours.

All control loops were running in the control system Procos II, and data acquisition was done on-line in the same system for all parameters. Off gas measurement for CO₂ and O₂ was done on-line by a photo-acoustic gas analyzer.

### Results

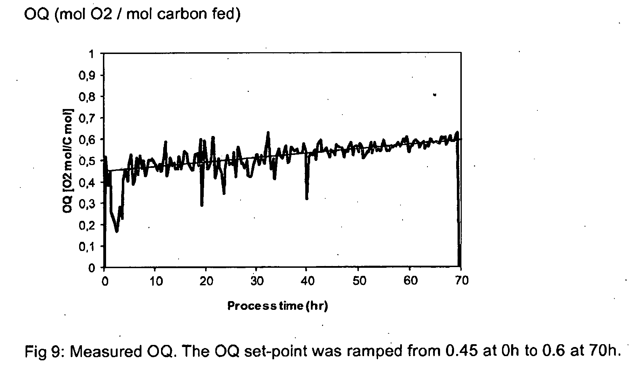

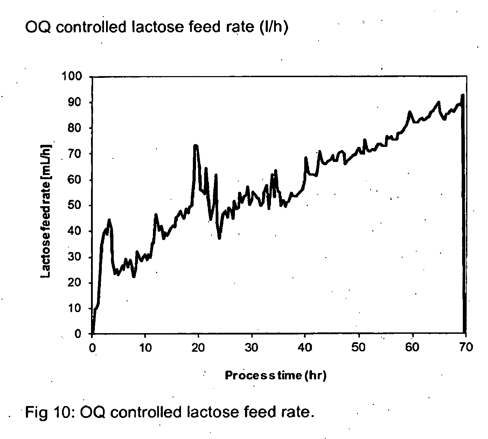

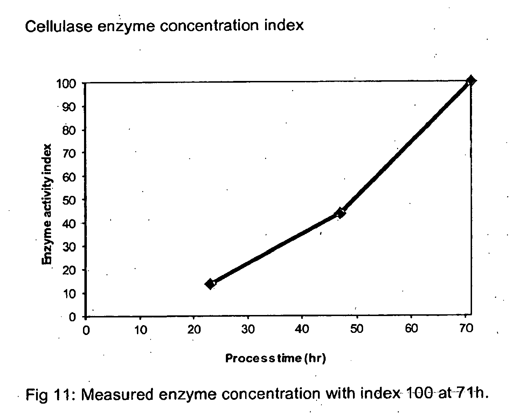

The main fermentation was successfully regulated by the OQ controller which increased the lactose feed rate from 0h to 70h (see Fig 9 and Fig 10). OQ was slightly below the set-point ramp until 4h, but thereafter it was maintained at the set-point ramp. Cellulase measurements confirm that enzyme was produced during the process - with index 100 at 71 h (see Fig 11).

### Conclusions

It has been shown that an OQ controller can automatically adjust the lactose feed rate according to the needs and limitations during fermentation of *Trichoderma reesei* and thereby maintaining a specified metabolic state.

## Claims

1. A method of controlling the rate of carbon addition to a fermentation medium in a fed batch or a continuous fermentation medium comprising
measuring the C-moles lost to carbon dioxide;
measuring the C-moles added via the carbon addition;
calculating the carbon dioxide feed quotient, FQ, as C-moles carbon dioxide produced divided by C-moles carbon added over a time period; and
adjusting the rate of carbon addition depending on the FQ of interest, or
measuring the moles of consumed oxygen;
measuring the C-moles added via the carbon addition;
calculating the oxygen feed quotient, OQ, as moles oxygen consumed divided by C-moles carbon added over a time period; and
adjusting the rate of carbon addition depending on the OQ of interest,
wherein said fermentation medium comprises a microorganism producing a compound of interest or wherein said fermentation medium comprises a microorganism producing a microorganism of interest.

2. The method according to claim 1, wherein the microorganism is a bacterium or a fungus.

3. The method according to claim 2, wherein the bacterium is a *Bacillus* strain.

4. The method according to claim 1, wherein the compound of interest is a protein or a peptide.

5. The method according to claim 1, wherein the protein is an enzyme.

6. The method according to claim 1, wherein the compound of interest is a polysaccharide.

7. The method according to claim 6, wherein the polysaccharide is hyaluronic acid.

8. The method according to claim 1, wherein the carbon source is selected from the group consisting of glucose, sucrose, lactose, glycerol and maltose.

9. The method according to claim 1, wherein the FQ is in the range of from 0.25 to 0.75.

10. The method according to claim 1, wherein the OQ is in the range of from 0.25 to 0.75.

11. The method according to claim 1, wherein the time period is from 1 second to 20 minutes.

12. The method according to claim 1, wherein the microorganism producing a microorganism of interest is a yeast.

13. The method according to claim 1, wherein the compound of interest is recovered.

14. The method according to claim 2, wherein the fungus is an *Aspergillus* strain or a *Trichoderma* strain.

## Patentansprüche

1. Verfahren zum Kontrollieren der Geschwindigkeit der Kohlenstoffzugabe zu einem Fermentationsmedium in einem Fed-batch- oder kontinuierlichen Fermentationsmedium, umfassend
Messen der C-Mol, die an Kohlenstoffdioxid verloren gehen;
Messen der C-Mol, die über Kohlenstoffzugabe zugefügt werden;
Berechnen des Kohlenstoffdioxid-Einspeisungsquotienten, FQ (carbon dioxide feed quotient), als C-Mol hergestelltes Kohlenstoffdioxid dividiert durch zugefügte C-Mol Kohlenstoff über einen Zeitraum; und
Einstellen der Geschwindigkeit der Kohlenstoffzugabe abhängig vom interessierenden FQ, oder
Messen der Mol an verbrauchtem Sauerstoff;
Messen der über die Kohlenstoffzugabe zugefügten C-Mol;
Berechnen des Sauerstoff-Einspeisungsquotienten, OQ (oxygen feed quotient), als Mol verbrauchter Sauerstoff dividiert durch C-Mol Kohlenstoff, die über einen Zeitraum zugefügt werden; und
Einstellen der Geschwindigkeit der Kohlenstoffzugabe abhängig vom interessierenden OQ,
wobei das Fermentationsmedium einen Mikroorganismus umfasst, der eine interessierende Verbindung herstellt, oder wobei das Fermentationsmedium einen Mikroorganismus umfasst, der einen interessierenden Mikroorganismus herstellt.

2. Verfahren nach Anspruch 1, wobei der Mikroorganismus ein Bakterium oder ein Pilz ist.

3. Verfahren nach Anspruch 2, wobei das Bakterium ein *Bacillus*-Stamm ist.

4. Verfahren nach Anspruch 1, wobei die interessierende Verbindung ein Protein oder ein Peptid ist.

5. Verfahren nach Anspruch 1, wobei das Protein ein Enzym ist.

6. Verfahren nach Anspruch 1, wobei die interessierende Verbindung ein Polysaccharid ist.

7. Verfahren nach Anspruch 6, wobei das Polysaccharid Hyaluronsäure ist.

8. Verfahren nach Anspruch 1, wobei die Kohlenstoffquelle aus der Gruppe bestehend aus Glucose, Saccharose, Lactose, Glycerin und Maltose ausgewählt ist.

9. Verfahren nach Anspruch 1, wobei der FQ im Bereich von 0,25 bis 0,75 liegt.

10. Verfahren nach Anspruch 1, wobei der OQ im Bereich von 0,25 bis 0,75 liegt.

11. Verfahren nach Anspruch 1, wobei die Zeitdauer von 1 Sekunde bis 20 Minuten beträgt.

12. Verfahren nach Anspruch 1, wobei der einen interessierenden Mikroorganismus herstellende Mikroorganismus eine Hefe ist.

13. Verfahren nach Anspruch 1, wobei die interessierende Verbindung gewonnen wird.

14. Verfahren nach Anspruch 2, wobei der Pilz ein *Aspergillus*-Stamm oder ein *Trichoderma*-Stamm ist.

## Revendications

1. Méthode permettant de contrôler la vitesse d'ajout du carbone à un milieu de fermentation dans un milieu de fermentation alimenté en mode discontinu ou continu comprenant la mesure des moles C perdues en dioxyde de carbone ;
la mesure des moles C ajoutées via l'ajout de carbone ;
le calcul du quotient d'alimentation en dioxyde de carbone, FQ, en moles C de dioxyde de carbone produites divisé par les moles C de carbone ajoutées sur une période de temps ; et
l'ajustement de la vitesse d'ajout du carbone en fonction du FQ d'intérêt, ou
la mesure des moles d'oxygène consommées ;
la mesure des moles C ajoutées via l'ajout de carbone ;
le calcul du quotient d'alimentation en oxygène, OQ, en moles d'oxygène consommées divisé par les moles C de carbone ajoutées sur une période de temps ; et
l'ajustement de la vitesse d'ajout du carbone en fonction du OQ d'intérêt,
dans laquelle ledit milieu de fermentation comprend un microorganisme produisant un composé d'intérêt ou dans laquelle ledit milieu de fermentation comprend un microorganisme produisant un microorganisme d'intérêt.

2. Méthode selon la revendication 1, dans laquelle le microorganisme est une bactérie ou un champignon.

3. Méthode selon la revendication 2, dans laquelle la bactérie est une souche de *Bacillus.*

4. Méthode selon la revendication 1, dans laquelle le composé d'intérêt est une protéine ou un peptide.

5. Méthode selon la revendication 1, dans laquelle la protéine est une enzyme.

6. Méthode selon la revendication 1, dans laquelle le composé d'intérêt est un polysaccharide.

7. Méthode selon la revendication 6, dans laquelle le polysaccharide est l'acide hyaluronique.

8. Méthode selon la revendication 1, dans laquelle la source de carbone est choisie dans le groupe constitué par le glucose, le saccharose, le lactose, le glycérol et le maltose.

9. Méthode selon la revendication 1, dans laquelle le FQ est dans la plage de 0,25 à 0,75.

10. Méthode selon la revendication 1, dans laquelle le OQ est dans la plage de 0,25 à 0,75.

11. Méthode selon la revendication 1, dans laquelle la période de temps est de 1 seconde à 20 minutes.

12. Méthode selon la revendication 1, dans laquelle le microorganisme produisant un microorganisme d'intérêt est une levure.

13. Méthode selon la revendication 1, dans laquelle le composé d'intérêt est récupéré.

14. Méthode selon la revendication 2, dans laquelle le champignon est une souche d'*Aspergillus* ou une souche de *Trichoderma*.
